# EUROPEAN PATENT SPECIFICATION

(11) **EP 2 665 489 B1**
(45) Date of publication and mention of the grant of the patent: **30.08.2017**
(21) Application number: 12704527.6
(22) Date of filing: 18.01.2012
(51) Int. Cl.: A61K 38/45, A61K 48/00, A61P 35/00, A61K 31/495, A61K 31/522, C12N 9/12, A61K 45/06

(54) **A COMBINATION COMPRISING AN ADENOVIRAL VECTOR HAVING A THYMIDINE KINASE GENE, GANCICLOVIR AND TEMOZOLOMIDE FOR USE IN THE TREATMENT OF GLIOBLASTOMA MULTIFORM**
EINE KOMBINATION ENTHALTEND EINEN ADENOVIRUSVEKTOR MIT EINEM THYMIDIN-KINASE GEN, GANCICLOVIR UND TEMOZOLOMID ZUR BEHANDLUNG VON GLIOBLASTOMA MULTIFORME
UNE COMBINATION COMPRENANT UN VECTEUR ADÉNOVIRAL AVEC UN GÈNE DE LA THYMIDINE KINASE, GANCICLOVIR ET TEMOZOLOMIDE POUR LE TRAITEMENT DU GLIOBLASTOME MULTIFORME

(30) Priority: 18.01.2011 GB 201100804
(43) Date of publication of application: 27.11.2013
(73) Proprietor: Gliotherapy Limited, Bladon Oxfordshire OX20 1FX (GB)
(72) Inventor: MAATTA, Ann-Marie, FIN-70210 Kuopio (FI); SAMARANAYAKE, Haritha, FIN-70210 Kuopio (FI); PIKKARAINEN, Jere, FIN-70210 Kuopio (FI); YLA-HERTTUALA, Seppo, FIN-70210 Kuopio (FI)
(74) Representative: Teuten, Andrew John
(86) International application number: PCT/GB2012/050108
(87) International publication number: WO 2012/098397

(56) References cited:
- WO-A1-00/28059
- RAINOV NIKOLAI G ET AL: "Temozolomide enhances herpes simplex virus thymidine kinase/ganciclovir therapy of malignant glioma", CANCER GENE THERAPY, vol. 8, no. 9, September 2001 (2001-09), pages 662-668, XP002675095, ISSN: 0929-1903 cited in the application
- DATABASE MEDLINE [Online] US NATIONAL LIBRARY OF MEDICINE (NLM), BETHESDA, MD, US; 20 September 2011 (2011-09-20), CHIOCCA E ANTONIO ET AL: "Phase IB study of gene-mediated cytotoxic immunotherapy adjuvant to up-front surgery and intensive timing radiation for malignant glioma.", XP002675096, Database accession no. NLM21844505 & CHIOCCA E ANTONIO ET AL: "Phase IB study of gene-mediated cytotoxic immunotherapy adjuvant to up-front surgery and intensive timing radiation for malignant glioma.", JOURNAL OF CLINICAL ONCOLOGY : OFFICIAL JOURNAL OF THE AMERICAN SOCIETY OF CLINICAL ONCOLOGY 20 SEP 2011 LNKD- PUBMED:21844505, vol. 29, no. 27, 20 September 2011 (2011-09-20), pages 3611-3619, ISSN: 1527-7755
- DATABASE MEDLINE [Online] US NATIONAL LIBRARY OF MEDICINE (NLM), BETHESDA, MD, US; 20 September 2011 (2011-09-20), CHIOCCA E ANTONIO ET AL: "Phase IB study of gene-mediated cytotoxic immunotherapy adjuvant to up-front surgery and intensive timing radiation for malignant glioma.", XP002675096, Database accession no. NLM21844505

## Description

### Field of Invention

This invention relates to a drug combination for the treatment of cancer or of a disease characterised by an impaired mismatch repair (MMR) pathway.

### Background of the Invention

Herpes simplex virus type 1, thymidine kinase (HSV-tk) gene therapy is based on the prodrug activating enzyme that converts a non-toxic compounds such as ganciclovir, (GCV) into a toxic metabolite. The cell destruction by HSV-tk/GCV is cell cycle dependent, where only dividing cells will be affected. This is of particular advantage in brain cancer gene therapy, where the rapidly dividing tumour cells are surrounded by non-dividing normal brain cells. Therapy by HSV-tk is disclosed in EP1135513.

Temozolomide (TMZ, imidazole tetrazinone) is an oral alkylating agent that can cross the blood brain barrier (BBB). Temozolomide is an oral alkylating agent that is a derivative of dacarbazine. TMZ undergoes spontaneous hydrolysis at physiological pH to its active form 3-methyl-(triazen-1-yl) imidazole-4 carboxyamide (MTIC). The primary mode of cytotoxicity is by adding a methyl group at O⁶-position of guanine (O⁶-mG).

O⁶-mG by itself is not toxic to the cells. However, O⁶-mGs will become cytotoxic as a result of repeated cycles of futile efforts at repair by mismatch repair (MMR) pathway. This will ultimately lead to DNA strand breaks. It is known that a functional MMR pathway is essential to make cells sensitive to TMZ, in the absence of an active MGMT repair pathway (which occurs in 50% of malignant gliomas). Furthermore, defects in the MMR pathway can contribute to almost 100-fold resistance to alkylating agents such as TMZ.

A paper by Rainov et al (Cancer Gene Therapy, Vol 8, No 9, 2001 :pp 662-668), reports some experiments on the combination of HSV-tk/GCV gene therapy and TMZ chemotherapy, but the data do not show any compelling evidence of synergy.

### Summary of the Invention

The present invention is based on the discovery that HSV-tk gene therapy increases the gene expression of key mismatch repair (MMR) pathway proteins, namely MSH2 and MLH1. This led to the finding that HSV-tk/GCV gene therapy sensitises cells to chemotherapeutic agents, such as temozolomide (TMZ).

A study designed by the inventors confirmed that a combination of vector/prodrug gene therapy (such as HSV-tk/GCV) and a cytotoxic agent, has much improved efficacy in certain diseases (cancer was tested, but it is believed that this applies to all diseases characterised by an impaired MMR pathway), when compared to the use of either of the components alone, i.e. chemotherapy or vector/prodrug gene therapy.

It was also found that the administration protocol of these components is key to the surprising technical effect observed in the invention, i.e. the synergy. The inventors have found that the upregulation of the MMR pathway by vector/prodrug gene therapy takes approximately 2 days, and lasts for a maximum of 7 days after stopping prodrug therapy. Therefore, in order to see synergy it is necessary to begin administering the cytotoxic agent no later than 7 days after finishing prodrug therapy.

Furthermore, when the condition to be treated is characterised by an impaired MMR pathway, it is believed that a therapeutic benefit may be achieved by administering only the vector/prodrug gene therapy.

In a first aspect, the present invention is characterised by a new dosage regimen. Therefore, according to a first aspect, the present invention is an agent comprising a vector having a functional gene, a prodrug which can be converted into a cytotoxic agent by an expression product of the gene, and another cytotoxic agent, as a combined preparation for simultaneous, sequential or separate use in the therapy of cancer or of a disease characterised by an impaired mismatch repair (MMR) pathway, wherein the dosage regimen comprises beginning prodrug therapy after the vector has been administered, and beginning the another cytotoxic agent therapy no later than 7 days after the prodrug therapy has finished.

According to a second aspect, the present invention is an agent comprising a vector having a functional gene, and a prodrug which can be converted into a cytotoxic agent by an expression product of the gene, as a combined preparation for simultaneous, sequential or separate use in the therapy of a disease characterised by an impaired mismatch repair (MMR) pathway.

According to a third aspect, a method of treating glioblastoma multiforme, comprises the steps of:
a. Diagnosing in a human patient glioblastoma multiforme;
b. Identifying in said patient at least one glioblastoma multiforme tumor;
c. Resectioning said glioblastoma multiforme tumor to remove at least part of said glioblastoma multiforme tumor and expose tumor bed tissue;
d. Administering to said tumor bed tissue an AdHSV-*tk* adenoviral vector having a gene coding for thymidine kinase, whereby said AdHSV-*tk* adenoviral vector transfects said tumor bed tissue and said tumor bed tissue expresses said gene coding for thymidine kinase;
e. Within about 5 to about 19 days after administering said adenoviral vector to said human patient, further administering to said human patient ganciclovir;
f. Administering to said human patient temozolomide *per os* or by intravenous infusion.
The invention is defined in the claims.

### Description of the Figures

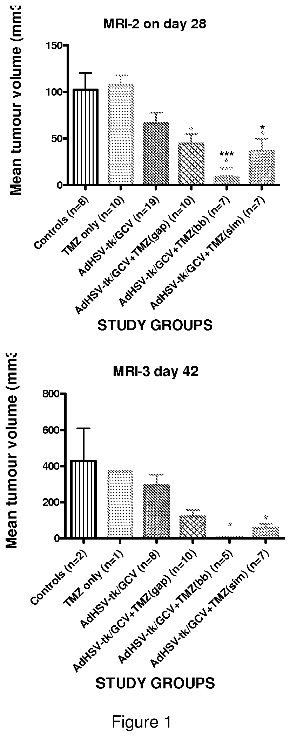

Figure 1 shows mean tumour volume at days 28 and 42 for different HSV-tk/GCV and TMZ/ dosage regimens.
Firgure 2 shows survival rate for different HSV-tk/GCV and TMZ dosage regimens.

### Description of the Preferred Embodiments

The present invention requires the administration of a vector having a functional gene, and a prodrug which can be converted by an expression product of that gene, into a cytotoxic agent. Preferably, the functional gene is a functional thymidine kinase gene. Preferably, the prodrug is ganciclovir or its analogues. It will be understood that the prodrug therapy should commence after the vector has been administered. Preferably the prodrug is administered from 5 to 19 days after administration of the vector.

Alternatively, suicide genes such as cytosine deminase, cytochrome P450, E coli purine nucleoside phosphorylase and carboxypeptidase G2, are suitable for use in the invention. Those suicide genes can be used in combination with suitable prodrugs, such as 5-fluorocytosine, cyclophosphamide, 6-methylepurine or F-araAMP or 4-benzoyl-L-glutamic acid (CMDA) or their chemical analogs, respectively. In one embodiment, the suicide gene, i.e. the vector, is cytosine deminase, and the prodrug is 5-fluorocytosine is suitable for use in the invention.

The vector is preferably locally administrated. When the therapy is of a cancerous tumour, for example, the vector may be administered directly into that cancerous tumour. Alternatively, it may be preferable to surgically remove the cancerous tumour, and then administer the vector into the wall of the tumour cavity.

As used herein, the term "wall of the tumour cavity" means the area of apparently healthy tissue (i.e. tissue which is apparently healthy to the eye of a surgeon) that remains once a tumour (or part of that tumour) is removed. Although the tissue is apparently healthy, it may contain malignant cells. The term "wall of the tumour cavity" refers to an area of non-tumour mass.

Preferably, the tumour resection is complete as possible, i.e. more than 90%, 95% or 98%. In a preferred embodiment, the vector is administered by injection approximately 1 cm (preferably between 0.5 cm and 5 cm, more preferably between 0.8 cm and 3 cm) deep into the wall of the tumour cavity. This ensures that the vector is into healthy tissue, i.e. is targeting primarily healthy cells (although it is appreciated that some malignant cells may reside in that area of apparently healthy tissue).

The vector that is used to transfer the gene may be any viral vector. However, it is preferred that it is derived from an adenovirus or a lentivirus. More preferably, it is derived from adenovirus.

The present invention is a combination therapy, comprising the administration of a gene therapy vector, a prodrug and a cytotoxic agent. The cytotoxic agent is preferably different from the cytotoxic agent that results from conversion of the prodrug (for example conversion of the ganciclovir), but otherwise the exact nature of the cytotoxic agent is not crucial, but it should preferably be a drug whose function is impaired by impaired MMR pathway. Some preferred cytotoxic agents are:
a) a chloroethylating agents such as carmustine, lomustine, fotemustine, nimustine, ranimustine or streptozocin;
b) a non-classical alkylating agent such as procarbazine;
c) a methylating triazine such as temozolomide, dacarbazine, altretamine, or mitobronitol;
d) a DNA cross-linking agent such as cisplatin, carboplatin, nedaplatin, oxaliplatin, triplatin, tetranitrate or satraplatin;
e) a topoisomerase II inhibitor such as doxorubicin, epirubicin, aclarubicin, daunorubicin, idarubicin, amrubicin, pirarubicin, valrubicin or zorubicin, mitoxantrone or pixantrone;
f) a topoisomerase I inhibitor such as topotecan, camptothesin, irinotecan, rubitecan or belotecan;
g) an anti metabolite (pyrmidine analogue) such as 5-FU, capecitabine, tegafur, carmofur, floxuridine or cytarabine;
h) an anti metabolite (purine analogue) such as 6-thioguanine or mercaptopurine; or
i) a cytotoxic DNA alkylating agent.

The most preferred cytotoxic agent is temozolomide (TMZ).

For synergy between vector/prodrug/cytotoxic, it is necessary for the MMR pathway to become upregulated, and therefore administration protocol/dosage regimen is key.

As used herein, "cytotoxic therapy" and "prodrug therapy" means the cytotoxic and prodrug dosage regimens, courses of treatment. Those therapies are for a specified period of time. The vector, however, need only be administered once.

Preferably, the another cytotoxic agent therapy begins no later than 7 days after prodrug therapy has finished. More preferably, the cytotoxic agent therapy begins no later than 6, 5, 4, 3, 2 or 1 day after prodrug therapy has finished. Preferably, the cytotoxic agent therapy begins less than 1 day after prodrug therapy finishes.

For the avoidance of doubt, included within the scope of the invention is both the situation where cytotoxic therapy is started immediately after prodrug therapy has finished, and also the situation where cytotoxic therapy is started before the prodrug therapy has finished (i.e. there is a period of simultaneous administration.

The cytotoxic therapy and the prodrug therapy may be started at the same time. Although, preferably, the cytotoxic agent therapy begins no earlier than 2 days after prodrug therapy begins. This allows for most efficient administration as the cytotoxic and prodrug are only combined once the MMR pathway has been upregulated. This is the most efficient dosage regimen.

Preferably, the prodrug therapy and the another cytotoxic agent therapy overlaps. More preferably, the therapies overlap for at least 3 days. More preferably, they overlap for at least 7, 10, 14 or 18 days.

Preferably, the prodrug therapy lasts for from 10 to 20 days. More preferably, it lasts for from 11 to 19, 12 to 18 or 13 to 17 days. Preferably, it lasts for 14 days.

In a preferred embodiment, the prodrug therapy begins from 2 to 5 days after vector administration (gene transfer). More preferably, the prodrug therapy begins at 5 days after gene transfer.

Preferably, the another cytotoxic therapy should begin at the earliest at 2 days after starting prodrug therapy, and at the latest at 7 days after stopping prodrug therapy.

The another cytotoxic agent therapy should begin no earlier than simultaneously with the commencement of prodrug therapy. It will be appreciated that it is preferred for the another cytotoxic agent therapy to begin no earlier than 2 days after commencement of prodrug therapy.

The upregulation of the MMR pathway is key to the invention. Therefore, it will be appreciated that the agent of the invention is useful in the treatment of a number of conditions. Examples of those conditions are cancer, actinic keratosis, pterygium diabetic retinopathy, atherosclerosis, asthma, chronic obstructive pulmonary disease, sarcoidosis, idiopathic pulmonary fibrosis, rheumatoid arthritis, pseudoexfoliation syndrome of the eye and Alzheimer's disease.

The most preferred therapy is of cancer. Preferably, the therapy is of a cancerous tumour, such as malignant glioma, or a tumour of the prostate. An agent of the invention may be used in the therapy of a cancer characterised by a normal or an impaired MMR pathway.

In a further preferred embodiment, an agent according to the present invention, when used to treat a cancerous tumour, also includes the administration of radiation. The radiation is preferably administered after the administration of the vector and the prodrug, and radiation therapy preferably starts at the same time as the cytotoxic chemotherapeutic agent (preferably, therapy is simultaneous).

The following study illustrates the present invention.

### Study

A study was conducted concerning tumour growth rate in a rat glioma model. There were 6 patient groups. Details of agents administered and the dosage regimen are shown in Table 1 below.

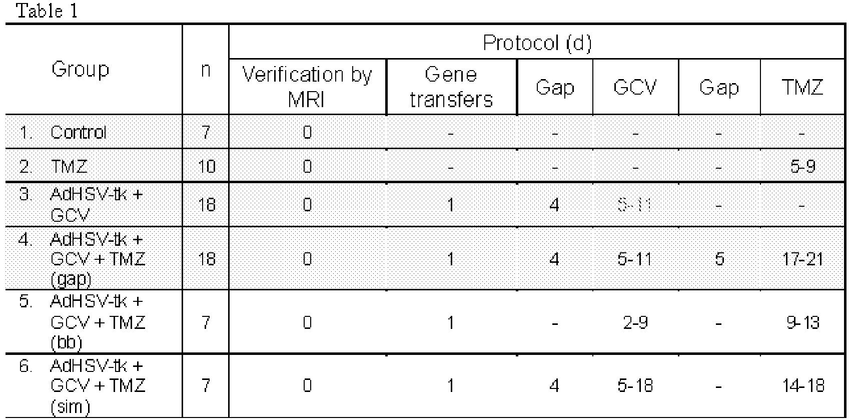

The results are shown in Figure 1. Group 5 shows the biggest decrease in tumour size.

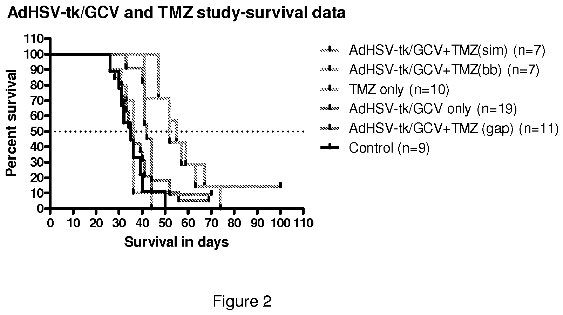

A second study was conducted in the rat glioma model concerning survival rates. The data (Figure 2) show that Group 6 had the longest survival rate, closely followed by group 5. This partly led the inventors to devise the dosage regimen of the invention (as slight overlap of prodrug/cytotoxic therapy is beneficial).

## Claims

1. An agent comprising an AdHSV-*tk* adenoviral vector having a functional thymidine kinase gene, ganciclovir as a prodrug which can be converted into a cytotoxic agent by an expression product of the gene, and temozolomide as another cytotoxic agent, as a combined preparation for simultaneous, sequential or separate use in the therapy of human glioblastoma multiforme tumor, wherein said glioblastoma multiforme tumor is resectioned to remove at least part of said tumor and the dosage regimen comprises beginning the another cytotoxic agent therapy after the viral vector therapy and at least part of said prodrug therapy has been administered and while gene expression of key mismatch repair proteins MSH2 and MLH1 is increased and beginning the another cytotoxic agent therapy no later than 7 days after the prodrug therapy has finished.

2. An agent for use according to claim 1, wherein the another cytotoxic agent therapy begins no earlier than 2 days after prodrug therapy begins.

3. An agent for use according to claim 1 or claim 2, wherein the prodrug therapy and the another cytotoxic agent therapy overlaps.

4. An agent for use according to claim 3, wherein the therapies overlap for at least 3 days.

5. An agent for use according to any preceding claim, wherein the prodrug therapy lasts for from 10 to 20 days.

6. An agent for use according to any of claims 1 to 5, wherein the vector is administered into the wall of the tumor cavity, preferably at a depth of approximately 1 cm.

7. An agent for use according to any of claims 1 to 6, which additionally includes the administration of radiation to the tumor.

8. A combination agent comprising an AdHSV-*tk* adenoviral vector having a functional thymidine kinase gene, and ganciclovir as a prodrug which can be converted into a cytotoxic agent by an expression product of the gene, and temozolomide as another cytotoxic agent, as a combined preparation for simultaneous, sequential or separate use in the therapy of a human glioblastoma multiforme tumor, wherein said viral vector and prodrug are administered in an amount sufficient to increase gene expression of key mismatch repair proteins MSH2 and MLH1 and wherein the another cytotoxic agent are administered while said expression is increased.

9. A combination agent for use according to claim 8, wherein said viral vector comprises an AdHSV-tk adenoviral vector having a gene coding for thymidine kinase.

10. A combination of an AdHSV-*tk* adenoviral vector, ganciclovir and temozomide for use in method of treating human glioblastoma multiforme, said method comprising the steps of:
a. Diagnosing in a human patient glioblastoma multiforme;
b. Identifying in said patient at least one glioblastoma multiforme tumor;
c. Resectioning said glioblastoma multiforme tumor to remove at least part of said glioblastoma multiforme tumor and expose tumor bed tissue;
d. Administering to said tumor bed tissue an AdHSV-*tk* adenoviral vector having a gene coding for thymidine kinase, whereby said AdHSV-*tk* adenoviral vector transfects said tumor bed tissue and said tumor bed tissue expresses said gene coding for thymidine kinase;
e. Within 5 days after administering said adenoviral vector to said human patient, further administering to said human patient ganciclovir;
f. Administering to said human patient temozolomide after said adenoviral vector *per os* or by intravenous infusion.

11. The combination for use of claim 10, wherein said glioblastoma multiforme is recurrent glioblastoma multiforme.

12. The combination for use of claims 10 or 11, wherein said temozolamide is administered in a plurality of 28-day cycles, each cycle comprising administration of a dose of 150 mg/m² per day each day for days 1 - 5 of said 28-day cycle, followed by a dose of 0 mg/m² per day for days 6 -28 of said 28-day cycle.

13. The combination for use of claim 12, wherein said plurality of 28-day cycles is preceded by period of 42 days wherein temozolamide is administered at a 30 dosage of 75 mg/m² per day.

14. The combination for use of any one of claims 10 to 13, wherein said administering of temozolomide is begun not earlier than the administering of ganciclovir.

## Patentansprüche

1. Agens, umfassend einen AdHSV-*tk*-Adenovirusvektor mit funktionsfähigem Thymidinkinasegen, Ganciclovir als Prodrug, das durch ein Expressionsprodukt des Gens in ein cytotoxisches Agens umgewandelt werden kann, sowie Temozolomid als weiteres cytotoxisches Agens, als Kombinationspräparat für die gleichzeitige, aufeinanderfolgende oder getrennte Verwendung in der Therapie von menschlichem Glioblastoma-multiforme-Tumor, wobei der Glioblastoma-multiforme-Tumor so resektiert wird, dass zumindest ein Teil des Tumors entfernt wird, und wobei das Dosierungsschema das Beginnen mit der Therapie mit dem anderen cytotoxischen Agens, nachdem die Virusvektortherapie und zumindest ein Teil der Prodrug-Therapie verabreicht worden ist und während die Genexpression der wichtigen "Mismatch Repair"-Proteine MSH2 und MLH1 erhöht ist, und Beginnen mit der Therapie mit dem anderen cytotoxischen Agens spätestens 7 Tage nach dem Ende der Prodrug-Therapie umfasst.

2. Agens nach Anspruch 1, wobei die Therapie mit dem anderen cytotoxischen Agens frühestens 2 Tage nach dem Beginn der Prodrug-Therapie beginnt.

3. Agens nach Anspruch 1 oder Anspruch 2, wobei sich die Prodrug-Therapie und die Therapie mit dem anderen cytotoxischen Agens überschneiden.

4. Agens nach Anspruch 3, wobei sich die Therapien mindestens 3 Tage lang überschneiden.

5. Agens nach einem vorhergehenden Anspruch, wobei die Prodrug-Therapie 10 bis 20 Tage lang dauert.

6. Agens nach einem der Ansprüche 1 bis 5, wobei der Vektor in die Wand der Tumorhöhle, vorzugsweise in einer Tiefe von etwa 1 cm, verabreicht wird.

7. Agens nach einem der Ansprüche 1 bis 6, das zusätzlich die Verabreichung von Strahlung an den Tumor beinhaltet.

8. Kombinationsagens umfassend AdHSV-*tk-*Adenovirusvektor mit funktionsfähigem Thymidinkinasegen, Ganciclovir als Prodrug, das durch ein Expressionsprodukt des Gens in ein cytotoxisches Agens umgewandelt werden kann, sowie Temozolomid als weiteres cytotoxisches Agens, als Kombinationspräparat für die gleichzeitige, aufeinanderfolgende oder getrennte Verwendung in der Therapie von menschlichem Glioblastoma-multiforme-Tumor, wobei der Virusvektor und das Prodrug in einer Menge verabreicht werden, die ausreicht, um die Genexpression der wichtigen "Mismatch Repair"-Proteine MSH2 und MLH1 zu erhöhen und wobei ein weiteres cytotoxisches Agens verabreicht wird, während die Expression erhöht ist.

9. Kombinationsagens nach Anspruch 8, wobei der Virusvektor einen AdHSV-*tk*-Adenovirusvektor mit einem Gen, das für Thymidinkinase codiert, umfasst.

10. Kombination von einem AdHSV-*tk*-Adenovirusvektor, Ganciclovir und Temozolomid zur Verwendung in einem Verfahren zur Behandlung von menschlichem Glioblastoma multiforme, wobei das Verfahren die folgenden Schritte umfasst:
a. Diagnostizieren von Glioblastome multiforme in einem menschlichen Patienten;
b. Identifizieren von mindestens einem Glioblastoma-multiforme-Tumor in dem Patienten;
c. Resektieren des Glioblastoma-multiforme-Tumors so, dass zumindest ein Teil des Glioblastoma-multiforme-Tumors entfernt wird und das Tumorbettgewebe freigelegt wird;
d. Verabreichen eines AdHSV-*tk*-Adenovirusvektors mit einem Gen, das für Thymidinkinase codiert, an das Tumorbettgewebe, wodurch der AdHSV-*tk*-Adenovirusvektor das Tumorbettgewebes transfiziert und das Tumorbettgewebe das Gen, das für Thymidinkinase codiert, exprimiert;
e. weiterhin Verabreichen von Ganciclovir an den menschlichen Patienten innerhalb von 5 Tagen nach der Verabreichung des Adenovirusvektors an den menschlichen Patienten;
f. Verabreichen von Temozolomid nach dem Adenovirusvektor an den menschlichen Patienten, und zwar entweder oral oder mittels intravenöser Infusion.

11. Kombination nach Anspruch 10, wobei es sich bei dem Glioblastoma multiforme um rekurrierendes Glioblastoma multiforme handelt.

12. Kombination nach den Ansprüchen 10 oder 11, wobei das Temozolomid in einer Vielzahl von 28-Tage-Zyklen verabreicht wird, wobei jeder Zyklus die Verabreichung einer Dosis von 150 mg/m² pro Tag täglich während der Tage 1-5 des 28-Tage-Zyklus, gefolgt von einer Dosis von 0 mg/m² pro Tag über die Tage 6-28 des 28-Tage-Zyklus umfasst.

13. Kombination nach Anspruch 12, wobei der Vielzahl von 28-Tage-Zyklen ein Zeitraum von 42 Tagen vorangeht, in dem Temozolomid in einer 30 Dosierung von 75 mg/m² pro Tag verabreicht wird.

14. Kombination nach Anspruch einem der Ansprüche 10 bis 13, wobei mit der Verabreichung von Temozolomid nicht vor der Verabreichung von Ganciclovir begonnen wird.

## Revendications

1. Agent comprenant un vecteur adénoviral AdHSV-*tk* comprenant un gène de thymidine kinase fonctionnelle, du ganciclovir en tant que promédicament qui peut être converti en un agent cytotoxique par un produit d'expression du gène, et du témozolomide en tant qu'autre agent cytotoxique, sous forme d'une préparation combinée pour utilisation simultanée, séquentielle ou séparée dans le traitement thérapeutique d'une tumeur de glioblastome multiforme humaine, où ladite tumeur de glioblastome multiforme est résectionnée pour retirer au moins une partie de ladite tumeur et la posologie comprend le démarrage du traitement thérapeutique par l'autre agent cytotoxique après le traitement thérapeutique par vecteur viral et au moins une partie dudit traitement thérapeutique par promédicament a été administrée, et pendant que l'expression génique des protéines de réparation des mésappariements clés MSH2 et MLH1 augmente, et le démarrage du traitement thérapeutique par l'autre agent cytotoxique pas plus de 7 jours après la fin du traitement thérapeutique par promédicament.

2. Agent selon la revendication 1, où le traitement thérapeutique par l'autre agent cytotoxique ne démarre pas moins de 2 jours après le traitement thérapeutique par promédicament.

3. Agent selon la revendication 1 ou la revendication 2, où le traitement thérapeutique par promédicament et le traitement thérapeutique par l'autre agent cytotoxique se superposent.

4. Agent selon la revendication 3, où les traitements thérapeutiques se superposent pendant au moins 3 jours.

5. Agent selon l'une quelconque des revendications précédentes, où le traitement thérapeutique par promédicament dure entre 10 et 20 jours.

6. Agent selon l'une quelconque des revendications 1 à 5, où le vecteur est administré dans la paroi de la cavité tumorale, préférentiellement à une profondeur d'environ 1 cm.

7. Agent selon l'une quelconque des revendications 1 à 6, qui inclut en outre l'administration de rayonnements au niveau de la tumeur.

8. Agent de combinaison comprenant un vecteur adénoviral AdHSV-*tk* présentant un gène de thymidine kinase fonctionnelle, et du ganciclovir en tant que promédicament qui peut être converti en un agent cytotoxique par un produit d'expression du gène, et du témozolomide en tant qu'autre agent cytotoxique, sous forme de préparation combinée pour utilisation simultanée, séquentielle ou séparée dans le traitement thérapeutique d'une tumeur de glioblastome multiforme humaine, où ledit vecteur viral et le promédicament sont administrés à une teneur suffisante pour augmenter l'expression génique des protéines de réparation des mésappariements clés MSH2 et MLH1 et où l'autre agent cytotoxique est administré lorsque ladite expression augmente.

9. Agent de combinaison selon la revendication 8, où ledit vecteur viral comprend un vecteur adénoviral AdHSV-*tk* présentant un gène codant la thymidine kinase.

10. Combinaison d'un vecteur adénoviral AdHSV-*tk*, de ganciclovir et de témozomide pour utilisation dans une méthode de traitement du glioblastome multiforme humain, ladite méthode comprenant les étapes suivantes :
a. Diagnostic chez un patient humain du glioblastome multiforme ;
b. Identification chez ledit patient d'au moins une tumeur de glioblastome multiforme ;
c. Résection de ladite tumeur de glioblastome multiforme pour retirer au moins une partie de ladite tumeur de glioblastome multiforme et exposer le tissu du lit tumoral ;
d. Administration audit tissu de lit tumoral d'un vecteur adénoviral AdHSV-*tk* présentant un gène codant la thymidine kinase, où ledit vecteur adénoviral AdHSV-*tk* transfecte ledit tissu de lit tumoral et ledit tissu de lit tumoral exprime ledit gène codant la thymidine kinase ;
e. Dans les 5 jours qui suivent l'administration dudit vecteur adénoviral audit patient humain, administration ultérieure audit patient humain de ganciclovir ;
f. Administration audit patient humain de témozolomide après ledit vecteur adénoviral *per os* ou par perfusion intraveineuse.

11. Combinaison selon la revendication 10, où ledit glioblastome multiforme est le glioblastome multiforme récurrent.

12. Combinaison selon les revendications 10 ou 11, où ledit témozolamide est administré en une multitude de cycles de 28 jours, chaque cycle comprenant l'administration d'une dose de 150 mg/m² par jour à chaque our pendant les ours 1 à 5 dudit cycle de 28 jours, suivie d'une dose de 0 mg/m² par jour pendant les jours 6 à 28 dudit cycle de 28 jours.

13. Combinaison selon la revendication 12, où ladite multitude de cycles de 28 jours est précédée d'une période de 42 jours, où le témozolamide est administré à une dose 30 de 75 mg/m² par jour.

14. Combinaison selon la revendication l'une quelconque des revendications 10 à 13, où ladite administration de témozolomide ne commence pas plus tôt que l'administration de ganciclovir.
